# EUROPEAN PATENT SPECIFICATION

(11) **EP 2 262 762 B1**
(45) Date of publication and mention of the grant of the patent: **22.07.2015**
(21) Application number: 09720196.6
(22) Date of filing: 09.03.2009
(51) Int. Cl.: C07C 229/38, C07C 215/00

(54) **CYCLOHEXANEDIMETHANAMINE BY DIRECT AMINATION OF CYCLOHEXANEDIMETHANOL**
CYCLOHEXANDIMETHANAMIN DURCH DIREKTE AMINIERUNG VON CYCLOHEXANDIMETHANOL
CYCLOHEXANE DIMÉTHANAMINE PAR AMINATION DIRECTE DU CYCLOHEXANE DIMÉTHANOL

(30) Priority: 10.03.2008 US 35234 P
(43) Date of publication of application: 22.12.2010
(73) Proprietor: Huntsman Petrochemical LLC, The Woodlands, TX 77380 (US)
(72) Inventor: RENKEN, Terry, L., Conroe, TX 77385 (US); KLEIN, Howard, P., Austin, TX 78759 (US)
(74) Representative: Swinnen, Anne-Marie
(86) International application number: PCT/US2009/036458
(87) International publication number: WO 2009/114438

(56) References cited:
- US-A- 3 143 570
- US-A- 5 789 620
- US-A- 5 973 208
- US-A- 6 008 384
- US-B1- 6 187 957

## Description

### BACKGROUND OF THE INVENTION

### Field of the Invention

The present invention relates generally to methods of producing amines and more particularly to the direct (one-step) amination of cyclohexanedimethanol to produce cyclohexanedimethanamine (CHDMA).

### Background of the Invention

Traditional methods of turning alcohols into amines involve multiple step reactions. One method to produce 1,3-CHDMA involves using a nitrile route starting with meta xylene, then hydrogenating that intermediate to meta-xylenediamine (MXDA), which is further hydrogenated to the 1,3-CHDMA. Another method involves producing a mixture of 1,3-CHDMA and 1,4-CHDMA via reduction of a mixture of 1,3- and 1,4- benzenedicarboxylic acids to the two aldehydes, then reductive amination to the mixture of 1,3- and 1,4-CHDMA. These methods require multiple steps that decrease manufacturing efficiency. Also the above methods may only allow certain types of cyclohexanedimethanamine to be produced.

US5789620 discloses to prepare 1,4-CHDMA by reacting 40 to 80 % aqueous solution of 1,4-cyclohexanedimethanol with a mixture of hydrogen and ammonia in the presence of a nickel/copper/chromium catalyst at a pressure of 100 to 250 bar and a temperature of 150 deg C to 250 deg C.

### BRIEF SUMMARY OF SOME OF THE PREFERRED EMBODIMENTS

Embodiments of the present invention disclose a method for producing a cyclohexanedimethanamine by contacting a catalyst with a pressurized amination mixture that includes a cyclohexanedimethanol, an amino compound, and a hydrogen.

### DETAILED DESCRIPTION OF THE PREFERRED EMBODIMENTS

Embodiments of the present invention disclose a method for producing a cyclohexanedimethanamine product by a reductive amination process. The method provides a cyclohexanedimethanol and contacts the cyclohexanedimethanol with an amino compound under a pressure that is greater than atmospheric pressure to form a pressurized amination mixture. The pressurized amination mixture contacts a catalyst.

The present invention includes a cyclohexanedimethanol, an amino compound, and a hydrogen source. The cyclohexanedimethanol may include 1,4-cyclohexanedimethanol, 1,3-cyclohexanedimethanol, the cis and trans versions thereof, and combinations thereof. One skilled in the art, with the benefit of this disclosure, will recognize appropriate cyclohexanedimethanols for use in this invention.

The amino compounds useful in this invention include any compounds that can contribute a reactive amine species. In embodiments of the present invention the amino compounds may include ammonia, organic amines, or combinations thereof. One skilled in the art with the benefit of this disclosure will recognize other appropriate amino compounds for use in this invention.

The reaction mixture also includes a hydrogen source. In one embodiment, hydrogen gas is used. Other compounds that can donate a reactive hydrogen species may be used. One skilled in the art, with the benefit of this disclosure will recognize other appropriate hydrogen sources for use in this invention.

The cyclohexanedimethanol, amino compound, and the hydrogen are contacted under a pressure that is greater than atmospheric pressure to form a pressurized amination mixture. In one embodiment, the pressurized amination mixture has a pressure in the range of 20.68bar (300 pounds per square inch (psi)) to 344.74 bar (5000 psi). In another embodiment, the pressurized amination mixture has a pressure of 124.11 bar (1800 psi) to 137.89 bar (2000 psi). One skilled in the art, with the benefit of this disclosure will recognize appropriate pressures to use in embodiments of the present invention.

In embodiments of the present invention, the temperature of the pressurized amination mixture has a temperature in the range of 150 degrees Celsius (°C) to 250 °C. In an embodiment, the pressurized amination mixture has a temperature of 200-°C. One skilled in the art, with the benefit of this disclosure will recognize appropriate temperatures for use in the present invention.

In embodiments of the present invention, the pressurized amination mixture described above is contacted with a catalyst. The catalysts useful in this invention include nickel, copper, tin, and zirconium, to catalyze the amination of alcohols. The catalysts may be unsupported or supported on materials such as alumina, silica, silica-alumina, zirconia, titania, carbon, and other known supports. The catalyst may be in tablet form. In another embodiment, the catalyst comprises nickel, copper, tin, and zirconium. One skilled in the art, with the benefit of this disclosure will recognize appropriate catalysts for use in embodiments of the present invention.

The catalysts of the present invention may also include promoters. Promoters generally include materials that enhance catalytic activity of a catalyst. Promoters may include chromium, iron, zinc, manganese, molybdenum, and combinations thereof.

The reaction of the pressurized amination mixture and the catalyst forms a cyclohexanedimethanamine product. As more fully described in the Examples below, the reductive amination process may create more than one chemical species. These species may include cyclohexanedimethanamine as well as a reaction effluent mixture of other side products which may include monoamine derivative(s) of cyclohexanedimethanol, unreacted diol(s), light bicyclic amines, and heavies.

In another embodiment of the present invention, the cyclohexanedimethanamine product is distilled. In an embodiment, the distillation includes separating the cyclohexanedimethanamine product from the reaction effluent mixture. This may also include separating the monoamine derivative(s) of cyclohexanedimethanol and unreacted diol(s) from other chemical species that are in present the reaction effluent mixture, such as light bicyclic amines and heavies. One skilled in the art will recognize that distillation and separating methods often do not result in complete purity of chemical species. Therefore distilled chemicals may still be tainted with minute amounts of other chemical species.

In another embodiment of the present invention, the monoamine derivative(s) of cyclohexanedimethanol and unreacted diol(s) are run through the amination process again to form the cyclohexanedimethanamine. This may be accomplished by recycling the recovered monoamine derivative(s) of cyclohexanedimethanol and unreacted diol(s) back into the method for producing a cyclohexanedimethanamine product. This method may include contacting fresh cyclohexanedimethanol with the amino compound and the recovered monoamine derivative(s) of cyclohexanedimethanol, recovered unreacted diol(s) and hydrogen under a pressure that is greater than atmospheric pressure to form a second pressurized amination mixture and contacting the second pressurized amination mixture with the catalyst.

The cyclohexanedimethanamine products produced by methods disclosed hereunder may be used in a variety of applications. For example, the 1,4-CHDMA is an aliphatic diamine useful in several polymer applications including without limitation, epoxy curing, polyamides, and polyureas. The diisocyanate derivative of 1,4-CHDMA is also useful in polyurethane and polyurea applications.

### EXAMPLES

In these Examples, 1,4-Cyclohexanedimethanamine (CHDMA), a mixture of the trans and cis isomers, was readily prepared in high selectivity by a one-step amination of 1,4-cyclohexanedimethanol with ammonia and a nickel-based reductive amination catalyst. This invention offers an attractive alternative to the multiple-step nitride route in the preparation of 1,4-CHDMA.

Reference Example 1. Amination of 1,4-Cyclohexanedimethanol. The amination was performed in a 100 cubic centimeter (cc) tubular reactor fully charged with 100 cc of Ni-Cu-Cr catalyst (Ni-2715, 1/8 diam x 1/8 inch pellets, 74% Ni, 12% Cu, 2% Cr). 1,4-cyclohexanedimethanol (CHDM-D, 99%; Eastman Chemicals Company, Kingsport, Tennessee), ammonia and hydrogen were each continuously fed to the heated reactor. The CHDM-D and ammonia feed rates were kept constant at 340.7 liter per hour (l/hr) and 794.9 (l/hr) (90 gallons per hour (g/hr) and 210 g/hr), respectively. The hydrogen was fed at rates of 1.7, 2.8, 5.6 and 11.1 liters/hour (l/hr) to observe any effects on conversion or selectivity. Samples were taken at reactor temperatures of 190 °C, 200 °C, 210 °C and 220 °C at each hydrogen rate. Reactor pressure was maintained at 172.37 barg (2500 pounds per square inch gauge (psig)) with a back pressure regulator. Reactor effluent samples were analyzed by gas chromatography and titrated for secondary amine content. Results are shown in Table 1 below. In Table 1: "3-Aza" refers to 3-azabicyclo[3.2.2]nonane; "Monoamine" refers to 1-hydroxymethyl-4-aminomethylcyclohexane; "Diamine" refers to 1,4-cyclohexanedimethanamine (1,4-CHDMA); "Residue" refers to any higher boiling materials than 1,4-CHDMA. Residue was determined by secondary amine content, subtracting the secondary amine contribution from 3-azabicyclo[3.2.2]nonane.

**Table 1. Amination of 1,4-Cyclohexanedimethanol**

| Run A: H₂ @ 2.8 l/hr | | | | |
|---|---|---|---|---|
| | | | | |
| Sample 6880-8- | 1A | 2A | 3A | 4A |
| Mid Rx Temp, °C | 181 | 190 | 201 | 209 |
| CHDM-D, g/hr | 90.0 | 88.0 | 92.0 | 86.0 |
| Ammonia, g/hr | 213.0 | 212.0 | 210.0 | 212.0 |
| % CHDM-D Conv | 60.9 | 80.5 | 90.1 | 95.1 |
| Wt% Selectivities: | | | | |
| 3-Aza | 0.81 | 1.01 | 1.53 | 1.52 |
| Diamine | 38.18 | 56.30 | 65.63 | 64.70 |
| Monoamine | 60.29 | 40.17 | 24.78 | 15.23 |
| Residue | 0.72 | 2.52 | 8.07 | 18.56 |
| | | | | |

| Run B: H₂ @ 5.6 l/hr | | | | |
|---|---|---|---|---|
| | | | | |
| Sample 6880-8- | 1B | 2B | 3B | 4B |
| Mid Rx Temp, °C | 180 | 190 | 201 | 210 |
| CHDM-D, g/hr | 88.0 | 87.0 | 86.0 | 87.0 |
| Ammonia, g/hr | 210.0 | 208.0 | 202.0 | 210.0 |
| % CHDM-D Conv | 47.2 | 73.8 | 90.0 | 96.0 |
| Wt% Selectivities: | | | | |
| 3-Aza | 0.61 | 0.94 | 1.70 | 2.55 |
| Diamine | 28.51 | 51.24 | 69.55 | 72.80 |
| Monoamine | 69.93 | 47.17 | 24.03 | 12.21 |
| Residue | 0.95 | 0.64 | 4.72 | 12.44 |
| | | | | |

| Run C: H₂ @ 1.71 l/hr | | | | |
|---|---|---|---|---|
| Sample 6880-8- | 1C | 2C | 3C | 4C |
| | | | | |
| Mid Rx Temp, °C | 181 | 190 | 201 | 211 |
| CHDM-D, g/hr | 92.0 | 87.0 | 92.0 | 96.0 |
| Ammonia, g/hr | 204.0 | 209.0 | 211.0 | 208.0 |
| % CHDM-D Conv | 45.6 | 62.8 | 76.2 | 91.7 |
| Wt% Selectivities: | | | | |
| 3-Aza | 0.32 | 0.61 | 1.14 | 2.19 |
| Diamine | 25.27 | 38.00 | 50.09 | 60.29 |
| Monoamine | 73.84 | 60.44 | 45.69 | 21.06 |
| Residue | 0.57 | 0.95 | 3.07 | 16.46 |

| Run D: H₂ @ 11.1 l/hr | | | | |
|---|---|---|---|---|
| Sample 6880-8- | 1D | 2D | 3D | 4D |
| Mid Rx Setpt, C | 180 | 191 | 201 | 210 |
| CHDM-D, g/hr | 88.0 | 88.0 | 86.0 | 86.0 |
| Ammonia, g/hr | 210.0 | 208.0 | 213.0 | 208.0 |
| % CHDM-D Conv | 44.0 | 70.6 | 87.4 | 95.9 |
| Wt% Selectivities: | | | | |
| 3-Aza | 0.65 | 1.05 | 1.82 | 2.66 |
| Diamine | 26.09 | 48.81 | 66.40 | 69.46 |
| Monoamine | 72.37 | 47.67 | 25.45 | 11.67 |
| Residue | 0.89 | 2.47 | 6.34 | 16.21 |

Reference Example 2. Isolation of 1,4-Cyclohexanedimethanamine by distillation of amination effluent. Reactor effluent was collected using the same reactor described in Example 1 and the following conditions: 340.7 l/hr (90 g/hr) CHDM-D, 794.9 l/hr (210 g/hr) ammonia, 5.6 1/hr hydrogen, 172.37 barg (2500 psig), 200 °C. Capillary GC analysis of the effluent was used to determine the following results as shown in Table 2.

**Table 2. Reactor Effluent Results**

| | | |
|---|---|---|
| | % CHDM-D conversion | 89.6 |
| | | |
| % Selectivities: | Cyclohexylmethylamine | 0.11 |
| | Cyclohexylmethanol | 0.07 |
| | 3-Azabicyclo[3.2.2] nonane | 1.54 |
| | Diamine | 69.00 |
| | Monoamine | 26.78 |
| | Residue | 2.48 |

A sample of the reactor effluent was fractionally distilled to afford pure diamine (bp 135 °C, 25 mm) and monoamine (167 °C, 25 mm).

While it has not yet been demonstrated, it is believed that the mixtures of CHDM-D diol and the monoamine intermediate can be fed to the amination reactor to produce diamine product. Thus, monoamine may be isolated and taken as a product or recycled back to the amination reactor to make diamine.

## Claims

1. A method for producing a cyclohexanedimethanamine product by a reductive amination process comprising the steps of:
a.- providing a cyclohexanedimethanol, an amino compound, and a hydrogen source;
b.- contacting the cyclohexanedimethanol with the amino compound and the hydrogen source under a pressure that is greater than atmospheric pressure to form a pressurized amination mixture; and
c.- contacting the pressurized amination mixture with a catalyst wherein the catalyst comprises nickel, copper, tin and zirconium.

2. The method of claim 1 wherein the catalyst further comprises a promoter.

3. The method of claim 1 wherein the catalyst further comprises a promoter, the promoter comprises chromium, iron, zinc, manganese, molybdenum, and combinations thereof.

4. The method of claim 1 wherein the cyclohexanedimethanol is selected from the group consisting of: 1,4-cyclohexanedimethanol, 1,3-cyclohexanedimethanol, and combinations thereof.

5. The method of claim 1 wherein the pressure that is greater than atmospheric pressure comprises a pressure in the range of 20.68 bar to 344.74 bar.

6. The method of claim 1 wherein the pressure that is greater than atmospheric pressure comprises a pressure in the range of 124.11 bar to 137.90 bar.

7. The method of claim 1 wherein the pressurized amination mixture has a temperature in the range of 150 °C to 250 °C.

8. The method of claim 1 wherein the pressurized amination mixture has a temperature of 200 °C.

9. The method of claim 1, further comprising the step of distilling the cyclohexanedimethanamine product.

10. The method of claim 9, wherein distilling the cyclohexanedimethanamine product comprises separating a reaction effluent mixture from the cyclohexanedimethanamine product.

11. The method of claim 10, wherein separating the reaction effluent mixture further comprises separating a monoamine derivative of cyclohexanedimethanol and an unreacted diol from the reaction effluent mixture.

12. The method of claim 11, further comprising the steps of:
a. contacting the cyclohexanedimethanol with the amino compound, the monoamine derivative of cyclohexanedimethanol, the unreacted diol and the hydrogen under a pressure that is greater than atmospheric pressure to form a second pressurized amination mixture; and
b. contacting the second pressurized amination mixture with the catalyst.

## Patentansprüche

1. Verfahren zur Herstellung eines Cyclohexandimethanamin-Produkts durch ein reduktives Aminierungsverfahren, umfassend die folgenden Schritte:
a. - Bereitstellen eines Cyclohexandimethanols, einer Aminoverbindung und einer Wasserstoffquelle;
b. - Kontaktieren des Cyclohexandimethanols mit der Aminoverbindung und der Wasserstoffquelle unter einem über dem atmosphärischen Druck liegenden Druck, um ein unter Druck gesetztes Aminierungsgemisch zu bilden; und
c. - Kontaktieren des unter Druck gesetzten Aminierungsgemisches mit einem Katalysator, wobei der Katalysator Nickel, Kupfer, Zinn und Zirkonium umfasst.

2. Verfahren nach Anspruch 1, wobei der Katalysator ferner einen Promotor umfasst.

3. Verfahren nach Anspruch 1, wobei der Katalysator ferner einen Promotor umfasst, wobei der Promotor Chrom, Eisen, Zink, Mangan, Molybdän und Kombinationen davon umfasst.

4. Verfahren nach Anspruch 1, wobei das Cyclohexandimethanol aus der Gruppe ausgewählt ist, die aus 1,4-Cyclohexandimethanol, 1,3-Cyclohexandimethanol und Kombinationen davon besteht.

5. Verfahren nach Anspruch 1, wobei der über dem atmosphärischen Druck liegende Druck einen Druck im Bereich von 20,68 bis 344,74 bar umfasst.

6. Verfahren nach Anspruch 1, wobei der über dem atmosphärischen Druck liegende Druck einen Druck im Bereich von 124,11 bis 137,90 bar umfasst.

7. Verfahren nach Anspruch 1, wobei das unter Druck gesetzte Aminierungsgemisch eine Temperatur im Bereich von 150 °C bis 250 °C aufweist.

8. Verfahren nach Anspruch 1, wobei das unter Druck gesetzte Aminierungsgemisch eine Temperatur von 200 °C aufweist.

9. Verfahren nach Anspruch 1, ferner umfassend den Schritt des Destillierens des Cyclohexandimethanamin-Produkts.

10. Verfahren nach Anspruch 9, wobei das Destillieren des Cyclohexandimethanamin-Produkts das Abtrennen eines Reaktionsabflussgemisches vom Cyclohexandimethanamin-Produkt umfasst.

11. Verfahren nach Anspruch 10, wobei das Abtrennen des Reaktionsabflussgemisches ferner das Abtrennen eines Monoaminderivats von Cyclohexandimethanol und eines nicht-umgesetzten Diols vom Reaktionsabflussgemisch umfasst.

12. Verfahren nach Anspruch 11, ferner umfassend die folgenden Schritte:
a. Kontaktieren des Cyclohexandimethanols mit der Aminoverbindung, dem Monoaminderivat von Cyclohexandimethanol, dem nicht-umgesetzten Diol und dem Wasserstoff unter einem über dem atmosphärischen Druck liegenden Druck, um ein zweites unter Druck gesetztes Aminierungsgemisch zu bilden; und
b. Kontaktieren des zweiten unter Druck gesetzten Aminierungsgemisches mit dem Katalysator.

## Revendications

1. Procédé pour préparer un produit consistant en cyclohexanediméthanamine par un procédé d'amination réductrice, comprenant les étapes de :
a.- fourniture d'un cyclohexanediméthanol, d'un composé à fonction amino et d'une source d'hydrogène ;
b.- mise en contact du cyclohexanediméthanol avec le composé à fonction amino et la source d'hydrogène sous une pression qui est supérieure à la pression atmosphérique pour former un mélange d'amination sous pression ; et
c.- mise en contact du mélange d'amination sous pression avec un catalyseur, le catalyseur comprenant du nickel, du cuivre, de l'étain et du zirconium.

2. Procédé suivant la revendication 1, dans lequel le catalyseur comprend en outre un promoteur.

3. Procédé suivant la revendication 1, dans lequel le catalyseur comprend en outre un promoteur, le promoteur comprenant du chrome, du fer, du zinc, du manganèse, du molybdène et leurs associations.

4. Procédé suivant la revendication 1, dans lequel le cyclohexanediméthanol est choisi dans le groupe consistant en : le 1,4-cyclohexanediméthanol, le 1,3-cyclohexane-diméthanol et leurs associations.

5. Procédé suivant la revendication 1, dans lequel la pression qui est supérieure à la pression atmosphérique comprend une pression dans l'intervalle de 20,68 bars à 344,74 bars.

6. Procédé suivant la revendication 1, dans lequel la pression qui est supérieure à la pression atmosphérique comprend une pression dans l'intervalle de 124,11 bars à 137,90 bars.

7. Procédé suivant la revendication 1, dans lequel le mélange d'amination sous pression est à une température dans l'intervalle de 150°C à 250°C.

8. Procédé suivant la revendication 1, dans lequel le mélange d'amination sous pression est à une température de 200°C.

9. Procédé suivant la revendication 1, comprenant en outre l'étape de distillation du produit consistant en cyclohexanediméthanamine.

10. Procédé suivant la revendication 9, dans lequel la distillation du produit consistant en cyclohexane-diméthanamine comprend la séparation d'un mélange d'effluent réactionnel du produit consistant en cyclohexanediméthanamine.

11. Procédé suivant la revendication 10, dans lequel la séparation du mélange d'effluent réactionnel comprend en outre la séparation d'un dérivé de monoamine de cyclohexane-diméthanol et d'un diol n'ayant pas réagi du mélange d'effluent réactionnel.

12. Procédé suivant la revendication 11, comprenant en outre les étapes de :
a- mise en contact du cyclohexanediméthanol avec le composé à fonction amino, le dérivé de monoamine de cyclohexanediméthanol, le diol n'ayant pas réagi et l'hydrogène sous une pression qui est supérieure à la pression atmosphérique pour former un second mélange d'amination sous pression ; et
b.- mise en contact du second mélange d'amination sous pression avec le catalyseur.
